Europäisches Patentamt

European Patent Office

Office européen des brevets

(19)

(11) **EP 1 379 556 B1**

(12) **EUROPEAN PATENT SPECIFICATION**

(45) Date of publication and mention
of the grant of the patent:
**20.12.2006 Bulletin 2006/51**

(51) Int Cl.:
*C07K 16/14* (2006.01)    *G01N 33/50* (2006.01)
*A61K 39/00* (2006.01)

(21) Application number: **02718674.1**

(22) Date of filing: **15.04.2002**

(86) International application number:
**PCT/KR2002/000685**

(87) International publication number:
**WO 2002/083737 (24.10.2002 Gazette 2002/43)**

(54) **ANTI PILYROSPORUM OVALE IGY AND ITS USES**

ANTI-PILYROSPORUM OVALE IGY UND DESSEN VERWENDUNG

ANTICORPS IGY DIRIGE CONTRE PITYROSPORUM OVALE ET SON UTILISATION

(84) Designated Contracting States:
**AT BE CH CY DE DK ES FI FR GB GR IE IT LI LU
MC NL PT SE TR**

(30) Priority: **14.04.2001 KR 2001020004**

(43) Date of publication of application:
**14.01.2004 Bulletin 2004/03**

(73) Proprietors:
• **Cotde, Inc.
Gangseo-gu,
Seoul 157-030 (KR)**
• **Lee & Joe Biotech Co.
Asan,
Choongnam 336-745 (KR)**

(72) Inventors:
• **LEE, Jong-Hwa
Asan-city,
Chungcheongnam-do 336-770 (KR)**
• **SAKONG, Jung
Boeun-kun,
Chungcheongbuk-do 370-882 (KR)**
• **JANG, Dong-Il
Anyang-city, Kyungki-do 430-708 (KR)**
• **YU, Chang-Seon
Yeonsu-ku,
Incheon-city 406-716 (KR)**
• **JANG, Ki-Ho,
104-804 1danji Jukong apt.
Yongin-city,
Kyungki-do 449-846 (KR)**
• **JUN, Jin-Hee
Namdong-ku,
Incheon-city 405-240 (KR)**

(74) Representative: **Winter, Brandl, Fürniss, Hübner
Röss, Kaiser,
Polte Partnerschaft Patent- und
Rechtsanwaltskanzlei
Alois-Steinecker-Strasse 22
85354 Freising (DE)**

(56) References cited:
**EP-A- 0 696 596        WO-A-01/19871**

• ZARGARI A ET AL: "IDENTIFICATION OF
ALLERGEN COMPONENTS OF THE
OPPORTUNISTIC YEAST PITYROSPORUM
ORBICULARE BY MONOCLONAL ANTIBODIES"
ALLERGY, MUNSKGAARD, COPENHAGEN, DK,
vol. 49, no. 1, January 1994 (1994-01), pages
50-56, XP001022533 ISSN: 0105-4538
• SCHMIDT M ET AL: "THE COMPLETE CDNA
SEQUENCE AND EXPRESSION OF THE FIRST
MAJOR ALLERGENIC PROTEIN OF
MALASSEZIA FURFUR, MAL F 1" EUROPEAN
JOURNAL OF BIOCHEMISTRY, BERLIN, DE, vol.
246, no. 1, 1997, pages 181-185, XP000876611
ISSN: 0014-2956
• NENOFF P ET AL: "[The yeast fungus Malassezia:
pathogen, pathogenesis and therapy]" DER
HAUTARZT; ZEITSCHRIFT FUR
DERMATOLOGIE, VENEROLOGIE, UND
VERWANDTE GEBIETE. JAN 2001, vol. 52, no. 1,
January 2001 (2001-01), pages 73-86,
XP002308723 ISSN: 0017-8470

EP 1 379 556 B1

- BÄCK ET AL: "Systemic ketoconazole for yeast allergic patients with atopic dermatitis." JOURNAL OF THE EUROPEAN ACADEMY OF DERMATOLOGY AND VENEREOLOGY : JEADV. JAN 2001, vol. 15, no. 1, January 2001 (2001-01), pages 34-38, XP002308724 ISSN: 0926-9959
- TINI M ET AL: "Generation and application of chicken egg-yolk antibodies" COMPARATIVE BIOCHEMISTRY AND PHYSIOLOGY. PAR A, MOLECULAR AND INTEGRATIVE PHYSIOLOGY, ELSEVIER SCIENCE, NEW YORK, NY, US, vol. 131A, no. 3, March 2002 (2002-03), pages 569-574, XP002300522 ISSN: 1095-6433
- DEVOS ET AL: 'The relevance of skin prick tests for Pityrosporum ovale in patients with head and neck dermatitis' ALLERGY vol. 55, no. 11, 2000, pages 1056 - 1058, XP002981632
- BERGBRANT ET AL: 'Seborrhoeic dermatitis and pityrosporum ovale: cultural, immunological and clinical studies' ACTA DERM VENEREOL SUPPL. vol. 67, 1991, STOCKHOLM, pages 1 - 36, XP002981656
- BERGBRANT ET AL: 'An immunological study in patients with seborrhoiec dermatitis' CLIN. EXP. DERMATOL. vol. 16, no. 5, 1991, pages 331 - 338, XP002981657
- BERGBRANT ET AL: 'The role of pityrosporum ovale in seborrheic dermatitis' SEMIN. DERMATOL. vol. 9, no. 4, 1990, pages 262 - 268, XP002981658
- ZARGARI ET AL: 'Cell surface expression of two major yeast allergens in the pityrosporum genus' CLIN. EXP. ALLERGY vol. 27, no. 5, 1997, pages 584 - 592, XP002981645

**Description**

BACKGROUND OF THE INVENTION

(a) Technological Field of the Invention

The present invention relates to a composition suitable for preventing dandruff, wherein said composition comprises a yolk antibody against Pityrosporum ovale which is the cause of dandruff. The antibody is produced by separating specific proteins from Pityrosporum ovale, immunizing animals with the proteins, and obtaining the anti-Pityrosporum ovale antibody from the animal.

(b) Description of the Related Arts

Pityrosporum ovale is a lipophilic yeast which is a member of normal flora on the normal human skin, especially on the scalp. Pityrosporum ovale is related to several skin diseases, in particular dandruff and seborrheic dermatitis. If the ratio of P.ovale to normal flora is 74% or more, dandruff symptom would manifest along with scale and cause itching. When the ratio is 83% or more, seborrheic dermatitis would break out (Shuster, S.: Br, J. Dematol, 3, 235 (1984), McGinley, K.J.et al: J Invest. Dermatol. 64, 40(1975)). In case there is an abnormal state of dandruff, the form of P. ovale is in mycelia and not in a form of germ spore , which makes it harder to be treated. Thus, the dandruff condition becomes chronic (Mycos 1997: 40 suppll: 29-32).

To improve the skin condition, products comprising antidandruff components such as Ketoconazole, Zinc Pyrithion, Climbazole, and others have been used. However, the components of these products have a low specificity to P. ovale, thereby killing useful microorganism as well as P. ovale. Therefore, it is highly likely that P. ovale may become a major microorganism in the scalp under seborrheic states.

There are several preceding examples to remove pathogenic microorganism with antibodies, especially egg yolk antibody. The typical examples are antibodies against acne germ, and bacteria inducing tooth decay (Korean Patent Laid-open Publication No. 1997-73607). In addition, Korean Patent Laid-Open Publication No. 2000-49499 discloses the preparation method of fermented milk using the subject yolk liquid maintaining anti-Helicobacter pylori antibody titer and intended for preventing general microorganisms and yeast from contamination. Also, the fermented milk is commercially available (KOREA YAKURT Co., Korea). However, no products or antibodies related with P. ovale have been introduced so far.

SUMMARY OF THE INVENTION

The present invention provides a composition suitable for preventing dandruff comprising a yolk antibody against Pityrosporum ovale as an effective agent, wherein the yolk antibody against Pityrosporum ovale is obtainable from eggs of an egg-laying hen immunized with Pityrosporum ovale as an antigen.

DETAILED DESCRIPTION OF THE INVENTION

The present invention is directed to a composition suitable for preventing dandruff, wherein said composition comprises a yolk antibody against Pityrosporum. The antibody is obtainable by separating specific proteins from Pityrosporum ovale, immunizing egg-laying hens with the proteins, and obtaining the antibody in the yolk.

Compared with the previous technologies, the present invention uses the preparation method of antibody against Pityrosporum ovale which is on the skin or scalp and usually causes and worsens dandruff, seborrheic dermatitis, and atopic dermatitis for the purpose of inhibiting proliferation of P. ovale or deactivating some enzymes secreted from the P. ovale. Since the antibody itself is a biological macromolecule isolated from the edible egg, it hardly has harmful effects compared with the chemical anti-fungal agent or hormone. Thus, it is a safe material for preventing and treating dandruff, seborrheic dermatitis, and atopic dermatitis.

According to the present invention, the preparation method of the anti-P. ovate antibody comprises the following four steps:

1. Preparing antigen from P.ovale;
2. Immunizing laying hens with the antigen to produce yolk including anti-Pityrosporum ovale IgY;
3. Isolating the IgY; and
4. Preparing a composition comprising the IgY against P. ovale.

The antibody of the present invention is obtainable by immunizing an egg-laying hen with an antigen (e.g. P.

ovale) and obtaining the antibody from its eggs. The method of injecting the antigen and obtaining the antibody is well known to a person skilled in the related field which the present invention pertains to.

In the preferred embodiment of the invention, the antigen can be crude extract or purified cell wall components of P. ovate. There was a report that P. ovale has unique proteins of 37KD and 67KD in cell wall which could not be found in other yeasts. Experimental results show that a monoclonal antibody against the unique proteins binds to P. ovale with binding activity of 90 % or more (Zargari.A et al., Clin Exp Allergy 1997 May 2795);pp.584-92). Therefore, the inventors of the present invention used the crude extract of P.ovale and components of the cell wall as an antigen.

In the blood of fowls, there are Immunoglobulins corresponding to mammalian IgG, IgM, and IgA in an amount of 5.0 mg, 1.25 mg, and 0.61 mg per ml of serum, respectively. The egg-laying fowls have characteristics of transferring the immune component to a chick through passive immunity. As a result, the antibody can be accumulated in the egg. Only IgG of the three kinds of Immunoglobulins remains in the egg yolk, and thus it is called as IgY (Immunoglobulin yolk). The concentration of IgY is about 25 mg per yolk ml which is known to be as an excellent polyclonal antibody.

In comparison with other methods to obtain an antibody from the mammals, there are important advantages in obtaining a specific antibody from the egg-laying hen. That is, a single egg can contain about 90 to 100 mg of antibody, which is comparable to the amount contained in blood extracted from a rabbit. It is easier to collect eggs from immunized chickens than extracting blood from mammals, and such collection of the eggs does not require sacrificing the animals. Furthermore, much more antibody can be produced because large scale breeding of immunized chickens is easily facilitated.

In particular, the IgY of the egg-laying hen can be produced in an amount of about 40 kg per year, which is as much as one hundred and twenty times of that of rabbit. Because of phylogenic difference between the fowls and the mammals, the IgY of fowls does not need to be cross-reacted with the mammal IgG, which is very useful to produce IgY specific to the antigen of the mammals. Above all, by using the egg-laying hen for producing antibody, it is possible to repetitively produce stable active antibody, whereby standardizing manufacturing processes, and researching and developing products would become possible.

The characteristics of the present invention lies in the use of eggs containing antibody that suppresses proliferation of P. ovale as effective component for preparing the composition useful for preventing dandruff.

The antigen according to the invention can be injected by an intramuscular, a hypodermic, an intravenous, abdominal or oral administration. Intramuscular injection is preferred. A dose of administrated antigen can be 10 μg or 1 mg in terms of protein amount in the antigen, and it depends on the condition or states of animals. The antigen is injected repetitively until the amount of antibody reaches a maximum value which can be examined by a shift of specific antibody amount in the yolk by ELISA (Enzyme-Linked Immunosorbent Assay) method.

The antibody according to the present invention can be isolated from the above eggs by ordinary methods for isolating antibody. The egg itself can be used as an antibody after drying and powdering, and it further comprises whole egg power, yolk powder, and water-soluble protein powder of egg yolk. The antibody could be purified from the egg yolk. The purified antibody could be used for preparing a pharmaceutical composition useful for preventing and treating the skin disease caused by P. ovale, especially anti-dandruff and seborrheic dermatitis.

To examine the effects of preventing and treating dandruff, seborrheic dermatitis and atopic dermatitis of anti-P. ovale antibody, the test was applied to someone who has skin diseases such as a dandruff or seborrheic dermatitis and atopic dermatitis with a shampoo and external formula. As a result, the yolk antibody against Pityrosporum ovale can be use as an effective component for preventing and treating dandruff and seborrheic dermatitis along for suppressing growth and activity of Pityrosporum species.

The following examples are intended only to illustrate the invention and are not intended to limit the scope of the invention as defined by the claims.

## Example 1: Culture of P. ovale

Pityrosporum ovale was cultured at 37°C for 3-4 days in the Brucella broth(Difco) in which antibiotic(Skirrow's supplement) and a small amount of sterillized oil was added.

## Example 2: Production of the antigen

P. ovale was purchased from KCTC (Korean Collection for Type Cultures) in the lyophilized form for making antigen. Pityrosporum ovale was cultured in the Brucella broth medium (Difco) in which antibiotics ( Skirrow's supplement) and a small amount of sterilized oil were supplemented. The cultured cells were harvested with centrifugal separation at 3000 x g and then re-suspended in phosphate buffer saline solution. After the cells were sonicated, the non-crushed cells were removed by centrifugation at 10,000 x g for 15 minutes. Then, the supernatant was taken and precipitated by ultra-centrifugation at 100,000 x g for 30 minutes. The precipitated cells were re-suspended in distilled water and used as an antigen for the following experiment.

**Example 3: Purification of cell wall components**

After the harvesting of cells which were cultured in Brucella broth medium according to Example 1, the cells were rinsed three to five times with normal saline solution and then were suspended in the saline solution. The cells were crushed completely by ultrasonic wave. Then, the cell liquid was filtered to separate insoluble component of cell wall with porous cellulose or nylon mesh or was precipitated with centrifugation according to Example 2. The filtrate or precipitate was used as an antigen for the following experiment.

**Example 4: Preparation of egg yolk containing antibody**

21 weeks-aged hens (Hy-Line Brown) that have already started to lay eggs were used for production of antibody. The antigens obtained in Examples 2 and 3 were mixed with Freund's complete adjuvant (Sigma chemical Co.) in the same amount and then emulsified. 1 ml of the emulsified antigen was intramuscularly injected into the leg of the hens and then boostered 3 times in six weeks.. Immunized eggs were then collected for testing the immunity titer and binding affinity to the antigen.

**Example 5: Test of the binding activity of the IgY**

To decide the effect of produced yolk antibody, ELISA method was used. The yolk antibody (IgY) to be used was purified by the following method.

5 ml of yolk-liquid was mixed with 5 ml of distilled water and added by 20 ml of 0.15% lambda Carageenan and was left at room temperature for 30 minutes. Then, the solution was centifugated at 20°C, 3,000 x g, for 15 minutes, and supernatant was taken. The supernatant was added by 19 % sodium sulfate (about 3g), stirred, left for 40 minutes at room temperature, and centrifuged at 10,000 x g for 15 minutes to produce precipitate. The precipitate was re-suspended in a phosphate buffer solution to obtain the antibody.

$1 \times 10^9$ cells/ml of P. ovale was poured into five 10 ml tubes, and the two types of antibodies in an amount of 12.5, 25, 50, and 100 mg/ml were added into four of the five tubes. The tube without antibodies was used as a control. The cells were shaken and cultured at 37 °C for 2 hours. Then, 1 ml of the culture solution was taken, heated at 60 °C for 30 minutes, and then kept cold.

The result of antibody reaction was measured by ELISA Titration method and is shown in Table 1.

As shown in Table 1, the IgY produced with cell wall component has higher binding activity. It was proved that the concentration of antibodies to reduce the cell number to half was 25 mg/ml.

Table 1: P, ovate cell number at various concentrations of IgY

| Inhibitory concentration (mg) | Cell concentration | |
|---|---|---|
| | IgY produced by cell wall | IgY produced by crude extract |
| 0 | $1.01 \times 10^9$ | $5.0 \times 10^9$ |
| 12.5 | $2.4 \times 10^7$ | $3.5 \times 10^7$ |
| 25 | $5.3 \times 10^4$ | $2.0 \times 10^5$ |
| 50 | $2.1 \times 10^3$ | $2.8 \times 10^3$ |

(continued)

| Inhibitory concentration (mg) | Cell concentration | |
|---|---|---|
| | IgY produced by cell wall | IgY produced by crude extract |
| 100 | . $1.8 \times 10^2$ | $1.2 \times 10^2$ |

ELISA Titration Method was performed by the following steps:

(1) Diluting P. ovale in each group with a coating buffer until the protein concentration reaches 5 $\mu$g/m$\ell$, 100$\mu\ell$ solution per well, pouring the diluted P. ovale at each well, and keeping it at 4°C for 48 hours;
(2) Adding 100$\mu$l of blocking buffer to the solution and reacting at 37°C for 2 hours;
(3) Rinsing with washing solution 3 times;
(4) Diluting IgY purified from egg yolk with blocking buffer to 100 $\mu$g/m$\ell$, pouring at 100$\mu\ell$ per well, and keeping at 4°C overnight or incubating at 37°C for 2 hours;
(5) Rinsing with washing solution 5 times;
(6) Diluting goat Anti-chicken IgY-AP Conjugate with blocking buffer to the ratio of 1:1000, pouring 100$\mu$l per well, and incubating at 37 °C for 2 hours;
(7) Adding 100 $\mu$l per well of the substrate and reacting in the dark room at 30 °C
(8) Rinsing with washing solution 6 times;
(9) Adding 100 $\mu$l of stop solution; and
(10) Reading by ELISA reader (450nm).

**Example 6: Purification of the produced IgY from the egg yolk**

The immunized eggs containing an anti-Pityrosporum ovale antibody were collected. 5 m$\ell$ of the egg yolk separated from the eggs was mixed with 5 m$\ell$ of distilled water and 20 m$\ell$ of 0.15% lambda carageenan and was left at room temperature for thirty minutes. After centrifugation at 3,000 x g for fifteen minutes at 20 °C, the supernatant was taken. Sodium sulfate was added to the supernatant to the final concentration of 19% and then was left at room temperature for four minutes. Then, the precipitate was obtained by centrifuging the resultant solution at 10,000 x g for fifteen minutes, and the solution was solubilized with phosphate buffer solution to produce the antibody. The protein concentration in total was measured with BCA protein assay reagent kit (Pierce). The IgY concentration was obtained by dividing measured absorbance at 280 nm with 1.33 of IgY absorption coefficient and was corrected with a standard curve of Chicken IgY (Promega).

**Example 7: Test for anti-dandruff effect of the purified egg yolk**

The anti-dandruff effect was tested by Skin Disc Diffusion Method. First of all, P. ovale was inoculated in an agar plate and then cultured at 37 °C for two days under aerobic conditions. The disc paper was sterilized with 70% ethanol, treated with ketoconazole, zincpyrithione, climbazol, salicylic acid, and anti-dandruff IgY in various concentration and was put on the agar plate. After incubating at 37°C for 48 hours, the size of inhibition zone was measured 3 times, and the mean size is shown in Table 2 where the inhibition zone shows the size of clear zone including the disc size (1.2 cm).

Table 2: The results from the Skin Disc Diffusion Method (n=3)

| Test sample | | Diameter of clear zone(cm) |
|---|---|---|
| Test material | Concentration(%) | |
| Ketoconazole | 0 | 0 |
| | 0.5 | 4.92 |
| | 1 | 5.06 |
| | 2 | 6.37 |

(continued)

| Test sample | | Diameter of clear zone(cm) |
|---|---|---|
| Test material | Concentration(%) | |
| Zinc pyrithione | 0 | 0 |
| | 0.25 | 4.83 |
| | 0.5 | 5.1 |
| | 1 | 5.1 |
| Climbazole | 0 | 0 |
| | 0.25 | 5.37 |
| | 0.5 | 5.57 |
| | 1 | 5.73 |
| Salicylic acid | 0 | 0 |
| | 0.75 | 0 |
| | 1.5 | 0 |
| | 3 | 1.4 |
| IgY | 0 | 0 |
| | 1 | 2.78 |
| | 2 | 4.83 |

According to Table 2, the diameter of clear zone of anti-P. ovale antibody is more than that of the known compounds. However, given that the molar concentration and the diffusion rate of IgY are low compared with those of the known compounds, IgY may be more effective.

**Examples 8 to 11: Anti-dandruff shampoo including egg yolk antibody**

The shampoo containing anti-P. ovale antibody was made and tested for the anti-dandruff effect.

Pyroctone amine, Zinc pyrithione, or IgY was dissolved in distilled water by adding sodium lauryl sulfate, Sodium Polyoxyethylenelauryl ether sulfate, diethanol lauryl amide, and propylene glycol in an amount as shown in Table 3 and by heating to 60°C. Then, the solution was cooled to 40°C, mixed with colors, para-benzoic acid ester, perfumes, and citric acid, and cooled to room temperature to produce the shampoo.

Table 3: Shampoo formula (units: percent by weight)

| Ingredients | Com. Exp 1 | Com. Exp 2 | Com. Exp 3 | Exp 8 | Exp 9 | Exp 10 | Exp 11 |
|---|---|---|---|---|---|---|---|
| Sodium lauryl sulfate | 20.0 | 20.0 | 20.0 | 20.0 | 20.0 | 20.0 | 20.0 |
| Sodium Polyoxyethylenelauryl ether sulfate | 20.0 | 20.0 | 20.0 | 20.0 | 20.0 | 20.0 | 20.0 |
| Diethanol Lauryl amide | 2.0 | 2.0 | 2.0 | 2.0 | 2.0 | 2.0 | 2.0 |
| Propylene glycol | 2.0 | 2.0 | 2.0 | 2.0 | 2.0 | 2.0 | 2.0 |
| Pyroctone amine | - | 0.5 | - | - | - | - | - |
| Zinc pyrithione | - | - | 0.5 | - | - | - | 0.5 |
| IgY | - | - | - | 1.0 | 5.0 | 10.0 | 1.0 |
| Colors | Little | Little | Little | Little | Little | Little | Little |
| Perfumes | Little | Little | Little | Little | Little | Little | Little |
| Citric acid | Little | Little | Little | Little | Little | Little | Little |

(continued)

| Ingredients | Com. Exp 1 | Com. Exp 2 | Com. Exp 3 | Exp 8 | Exp 9 | Exp 10 | Exp 11 |
|---|---|---|---|---|---|---|---|
| Parabenzoic acid ester | 0.2 | 0.2 | 0.2 | 0.2 | 0.2 | 0.2 | 0.2 |
| Distilled water | Add to 100 wt% | | | | | | |
| *Note: The egg yolk antibody is produced by purifying according to Example 6. | | | | | | | |

**Comparative Examples 1 to 3: Anti-dandruff shampoo containing egg yolk antibody**

To compare the effect of antidandruff shampoo using egg yolk antibody against Pityrosporum ovale, the shampoo composition containing the known compounds was shown in Table 3.

**Experiment 1: Clinical Test for Antidandruff effect**

Antidandruff and itching effect of the shampoos prepared- by the methods of Examples 8-11 and comparative examples 1-3 were tested as below.
The shampoos were applied to forty-nine male and female volunteers who have problems with dandruff for one month. After the volunteers were shampooed with a commonly-used shampoo product, the dandruff accumulated in three days was taken and weighed. The same volunteers were shampooed with shampoo product as shown in Table 2 every other day for 1 month, and the dandruff accumulated in three days was taken and weighed. The accumulated dandruff was taken by vacuum suction device from the scalp.
The reduction rate of dandruff was calculated as below.

$$\text{dandruff reduction rate(\%)} = \text{(the weight of dandruff before experiment}$$
$$\text{(mg)- the weight of dandruff after 1 month(mg))/ the weight of dandruff before}$$
$$\text{experiment} \times 100$$

The test results were shown in Table 4.

Table 4

| Shampoo | Average reduction rate | Answer (Anti-dandruff effect)(unit: person) | | |
|---|---|---|---|---|
| | | Yes | No | Unknown |
| Com. Exp 1 | 5.1 | - | 7 | - |
| Com. Exp 2 | 45.0 | 5 | 1 | 1 |
| Com. Exp 3 | 55.7 | 5 | - | 2 |
| Exp 8 | 43.7 | 6 | - | 1 |
| Exp 9 | 56.0 | 7 | - | - |
| Exp 10 | 58.2 | 7 | - | - |
| Exp 11 | 58.2 | 5 | - | 2 |

**Experiment 2: Antidandruff effect for microbiological experiment**

The shampoo materials in Table 3 were added to the Ym broth containing corn oil in amounts of 5.00, 1.00, 0.50, 0.10, 0.05, 0.01, and 0.005 % by weight, respectively to produce culture medium. $10^5$-$10^6$CFU (Colony Forming Unit)/ml of P. ovale were inoculated in the culture medium and cultured for two days. The Minimal Inhibitory Concentration (MIC) was obtained from the culture. The test results are shown in Table 5.

[Table 5]

|  | MIC (%) |
| --- | --- |
| Com Exp. 1 | 0.5-1 |
| Com Exp. 2 | 0.05-0.1 |
| Com Exp. 3 | 0.05-0.1 |
| Example 8 | 0.01-0.05 |
| Example 9 | 0.005-0.01 |
| Example 10 | 0.005 or less |
| Example 11 | 0.005 or less |

As shown from the above examples, the present invention provides an antidandruff composition comprising anti-P. ovale antibody which was produced by immunizing egg-laying hens with P. ovale as an antigen, and has an activity of suppression of the proliferation of P. ovale.

## Claims

1. A composition suitable for preventing dandruff comprising a yolk antibody against *Pityrosporum ovale* as an effective agent, wherein the yolk antibody against *Pityrosporum ovale* is obtainable from eggs of an egg-laying hen immunized with *Pityrosporum ovale* as an antigen.

2. The composition according to claim 1, wherein the antigen is crude extracts or the cell wall component of *Pityrosporum ovale*.

3. The composition according to claim 1, wherein the antigen is a 37kD protein or a 67kD protein which is specific to *Pityrosporum ovale* and exists on the surface of cell wall of *Pityrosporum ovale*.

## Patentansprüche

1. Zusammensetzung, die sich zum Verhindern von Schuppen eignet, umfassend einen Eigelbantikörper gegen *Pityrosporum ovale* als Wirkstoff, wobei der Eigelbantikörper gegen *Pityrosporum ovale* aus Eiern einer mit *Pityrosporum ovale* als Antigen immunisierten Legehenne erhältlich ist.

2. Zusammensetzung nach Anspruch 1, wobei das Antigen Rohextrakt oder Zellwandkomponente von *Pityrosporum ovale* ist.

3. Zusammensetzung nach Anspruch 1, wobei das Antigen ein 37 kD-Protein oder ein 67 kD-Protein ist, das für *Pityrosporum ovale* spezifisch ist und sich auf der Zellwandoberfläche von *Pityrosporum ovale* befindet.

## Revendications

1. Composition appropriée pour la prévention des pellicules comprenant un anticorps de jaune d'oeuf dirigé contre *Pityrosporum ovale* en tant qu'un agent efficace, dans laquelle l'anticorps de jaune d'oeuf dirigé contre *Pityrosporum ovale* est obtenu à partir des oeufs d'une poule pondeuse immunisée avec *Pityrosporum ovale* comme antigène.

2. Composition selon la revendication 1, dans laquelle l'antigène est un extrait brut ou le composant de la paroi cellulaire de *Pityrosporum ovale*.

3. Composition selon la revendication 1, dans laquelle l'antigène est une protéine de 37 kDa ou une protéine de 67 kDa qui est spécifique de *Pityrosporum ovale* et existe sur la surface de la paroi cellulaire de *Pityrosporum ovale*.